# EUROPEAN PATENT SPECIFICATION

(11) **EP 3 108 901 B1**
(45) Date of publication and mention of the grant of the patent: **17.03.2021**
(21) Application number: 15752671.6
(22) Date of filing: 17.02.2015
(51) Int. Cl.: A61K 51/08, A61K 51/04, A61K 49/06, A61K 51/02, A61P 9/10, A61K 103/00, A61K 103/10

(54) **COMPOSITION FOR IMAGING ATHEROSCLEROSIS AND METHOD FOR DIAGNOSING ATHEROSCLEROSIS BY USING SAME**
ZUSAMMENSETZUNG ZUR BILDGEBUNG VON ATHEROSKLEROSE UND VERFAHREN ZUR DIAGNOSE VON ATHEROSKLEROSE DAMIT
COMPOSITION POUR L'IMAGERIE DE L'ATHÉROSCLÉROSE ET PROCÉDÉ POUR DIAGNOSTIQUER L'ATHÉROSCLÉROSE EN UTILISANT CELLE-CI

(30) Priority: 21.02.2014 KR 20140020544
(43) Date of publication of application: 28.12.2016
(73) Proprietor: Korea University Research and Business Foundation, Seoul 136-701 (KR); Seoul National University R&DB Foundation, Seoul 08826 (KR)
(72) Inventor: SEO, Hong Seog, Seoul 140-011 (KR); KIM, Sungeun, Seoul 135-500 (KR); KIM, Eung Ju, Seoul 151-860 (KR); JEONG, Jae Min, Seoul 137-040 (KR)
(74) Representative: V.O.
(86) International application number: PCT/KR2015/001638
(87) International publication number: WO 2015/126164

(56) References cited:
- EP-A2- 2 671 841
- WO-A1-2010/087612
- JP-A- H08 508 488
- JP-A- 2006 526 642
- KR-A- 20100 087 511
- KR-B1- 100 557 008
- JOHANNA HAUKKALA ET AL: "68Ga-DOTA-RGD peptide: biodistribution and binding into atherosclerotic plaques in mice", EUROPEAN JOURNAL OF NUCLEAR MEDICINE AND MOLECULAR IMAGING, SPRINGER, BERLIN, DE, vol. 36, no. 12, 23 July 2009 (2009-07-23) , pages 2058-2067, XP019757623, ISSN: 1619-7089, DOI: 10.1007/S00259-009-1220-Z
- JAE YEON CHOI ET AL: "Development ofGa-labeled mannosylated human serum albumin (MSA) as a lymph node imaging agent for positron emission tomography", NUCLEAR MEDICINE AND BIOLOGY, ELSEVIER, NY, US, vol. 38, no. 3, 29 September 2010 (2010-09-29), pages 371-379, XP028190375, ISSN: 0969-8051, DOI: 10.1016/J.NUCMEDBIO.2010.09.010 [retrieved on 2010-10-08]
- IRINA VELIKYAN: 'Prospective of 68Ga-Radiophamaceutical Development' THERANOSTICS vol. 4, no. 1, 10 December 2013, ISSN 1838-7640 pages 47 - 80, XP055221217

## Description

### Technical Field

The present disclosure relates to a composition for imaging atherosclerosis and a method for diagnosing atherosclerosis using the same.

### Background Art

Atherosclerosis refers to a disease in which cholesterol is deposited on the inner portion (intima) of a blood vessel and intimal cells proliferate there, leading to narrowing or blocking of the blood vessel and decreased blood flow to the periphery. More specifically, atherosclerosis is a vascular disease in which, just as an aged water pipe becomes narrower in diameter as rust and impurities are deposited, cholesterol penetrates into intimal layer of a blood vessel and intimal cells and macrophages proliferate there, leading to the formation of an atheroma. The core of the atheroma is composed of acellular lipid core and it is covered by a hard fibrous plaque. When the plaque is unstable, it ruptures and forms blood clots in the blood vessel. Hemorrhage into the atheroma leads to rapid narrowing or even blocking of the blood vessel, thereby leading to restricted blood circulation to the periphery.

Methods for monitoring the occurrence and development of atherosclerosis include molecular imaging using PET/CT. For diagnosis of atherosclerosis by molecular imaging using PET/CT, fluorine-18 fluorodeoxyglucose (F18-FDG) has been used. The principle of diagnosis of atherosclerosis using F18-FDG is based on the increased uptake of glucose and FDG by foam cells.

However, the diagnostic method of imaging atherosclerosis using F18-FDG has some problems. Firstly, because FDG is a glucose analog, it is limited in controlling the body condition for diagnosis because blood glucose or metabolism-related hormones may be affected. Consequently, the accuracy of atherosclerosis diagnosis is decreased. In addition, the method is limited for high-risk groups such as those with diabetes because fasting or glycemic control is necessary. Secondly, although atherosclerosis occurs frequently in the brain and heart, it is difficult to detect atherosclerosis using F18-FDG because they utilize blood sugar for energy. Thirdly, manufacturing cost is excessively high because expensive equipment such as cyclotron is necessary to produce F18-FDG.

Korean Patent Registration No. 10-1351411 (patent document 1) and Korean Patent Registration No. 10-1055700 (patent document 2) disclose technologies related with the present disclosure. Specifically, the patent document 1 relates to a method of selectively diagnosing malignant tumors by distinguishing malignant tumors from inflammatory lesions in F18-FDG positron emission tomography, and the patent document 2 relates to mannosylated albumin labeled with Ga-68.

J. Haukkala et al (European Journal of Nuclear Medicine and Molecular Imaging, 2009, vol. 36, no. 12, pages 2058-2067) observed that ⁶⁸Ga-DOTA-RGD is accumulated into the plaques of atherosclerotic mice.

WO 2010/087612 A1 describes a conjugate of bifunctional chelating agents and mannosyl human serum albumin and its radioisotope labeled compounds for imaging immune system.

EP 2 671 841 A2 describes a nanoparticle coated with a ligand that may be used as an imaging agent to detect angiogenesis, cancer cells, immunocyte, hepatocyte, apoptosis or genes.

Jae Yeon Choi et al (Nuclear Medicine and Biology, vol. 38, no. 3, 29 September 2010, pages 371-379) describes the synthesis of a mannosylated human serum albumin (⁶⁸Ga-NOTA-MSA) as a lymph node imaging agent for positron emission tomography.

### Disclosure

### Technical Problem

The present disclosure is directed to providing a composition for imaging atherosclerosis, which shows high accuracy for atherosclerosis diagnosis, enables diagnosis of atherosclerosis even for a person with a disease such as diabetes and enables effective detection even for atherosclerosis occurring in the brain and heart. The present disclosure is also directed to providing a composition for imaging atherosclerosis at low manufacturing cost and providing a method for diagnosing atherosclerosis using the same.

### Technical Solution

In an aspect, the present disclosure provides a composition for use in a method of in vivo diagnosis of atherosclerosis, which composition comprises a radioisotope-labeled compound which is a bifunctional chelating agent-mannosylated human serum albumin, labeled with a radioisotope.

In another aspect, the present disclosure provides a method for imaging atherosclerosis using the composition for imaging atherosclerosis, which composition comprises a radioisotope-labeled compound which is a bifunctional chelating agent-mannosylated human serum albumin, labeled with a radioisotope..

### Advantageous Effects

A composition for imaging atherosclerosis according to the present disclosure shows high accuracy of diagnosis for atherosclerosis, enables diagnosis of atherosclerosis even for a person with diseases accompanied by metabolic problems of blood sugar such as diabetes and enables effective diagnosis even for atherosclerosis occurring in the brain and heart. In addition, manufacturing cost is low compared with the existing imaging composition for diagnosis of atherosclerosis. Therefore, atherosclerosis can be effectively diagnosed by using the same.

### Brief Description of Drawings

Fig. 1 and Fig. 2 show a result of measuring ⁶⁸GaCl labeling efficiency by TLC.
Fig. 3 shows a result of verifying stability by measuring radiochemical purity.
Fig. 4 shows specific binding of MSA-RITC to the mannose receptor in rat macrophages.
Fig. 5 shows a result of conducting positron emission tomography after intravenously injecting 1 mCi of ⁶⁸Ga-MSA to rabbit.
Fig. 6 shows RITC-labeled MSA detected at the atherosclerotic lesion of the aorta of rabbit with high cholesterol diet.
Fig. 7, Fig. 8 and Fig. 9 compare the result of imaging atherosclerosis using ¹⁸F-FDG of Comparative Example and ⁶⁸Ga-MSA of Example and Comparative Example.
Fig. 10 shows a result of comparing SUV in the brain parenchyma for Example and Comparative Example.
Fig. 11, Fig. 12 and Fig. 13 show a result of testing clinical applicability to a carotid atherosclerotic site for Example. ⁶⁸Ga-MSA images, SUVs, etc. were compared for a normal person and a patient with myocardial infarction.

### Best Mode

The inventors of the present disclosure have made consistent efforts to develop a method for imaging atherosclerosis, which shows high accuracy for diagnosis, enables diagnosis of atherosclerosis even for a person with a disease such as diabetes and is applicable even to lesions in the brain and heart. As a result, they have developed a method for imaging atherosclerosis according to the present disclosure.

In general, F18-FDG (¹⁸F-FDG) has been used in an imaging composition for diagnosing the occurrence and development of atherosclerosis. However, it is problematic in that accuracy is low because FDG is a glucose analog, it is difficult to be applied to a person with a disease such as diabetes and it is difficult to be applied to the brain and heart where the occurrence of atherosclerosis is the most important problem. In addition, the manufacturing cost of F18-FDG is high.

Specifically, a pharmaceutical composition used for imaging atherosclerosis according to the present disclosure contains a radioisotope-labeled compound which is a bifunctional chelating agent-mannosylated human serum albumin.

In the composition, the bifunctional chelating agent serves to bind to the radioisotope, the mannose group serves to bind to the mannose receptor, and the human serum albumin serves as a carrier/support for binding the bifunctional chelating agent to the mannose. The composition is desired to have a size of 1-100 nm, so that it is dispersed well in the blood and can move through a blood vessel. Because the bifunctional chelating agent and the mannose have a size of only about 0.5 nm, the carrier/support, i.e., the human serum albumin accounts for most of its size. The human serum albumin is ideal in size because it has a shape of a rugby ball with a major axis of 6 nm and a minor axis of 4 nm.

That is to say, the pharmaceutical composition for imaging atherosclerosis, wherein the mannosylated human albumin (MSA)is bound to the mannose which is a ligand of the mannose receptor and then one or more radioisotope selected from a group consisting of ⁶⁸Ga, ^{99m}Tc, ¹¹¹In, ¹⁸F, ¹¹C, ¹²³I, ¹²⁴I and ¹³¹I is attached thereto, enables molecular imaging of atherosclerosis by detecting the radioisotope and, through this, diagnosis of the occurrence and development of atherosclerosis. The mannose receptor is one of the cell membrane receptors present on foam cells occurring in atherosclerosis.

The radioisotope may be metallic. The radioisotope may be specifically one or more selected from a group consisting of ⁶⁸Ga, ^{99m}Tc, ¹¹¹In, ¹⁸F, ¹¹C, ¹²³I, ¹²⁴I and ¹³¹I, most specifically ⁶⁸Ga.

Because the composition is free from metabolic limitation, fasting is not necessary. And, because it is unrelated with metabolism-related hormones, it is applicable even to a person with diseases such as diabetes. In addition, it can also be used for the brain and heart, unlike the existing F18-FDG. Also, the manufacturing cost is low because no complicated or expensive equipment is required unlike F18-FDG. Conventionally, the expensive equipment called a cyclotron has been used to prepare F18-FDG. However, when ⁶⁸Ga is used as a metallic radioisotope as in the present disclosure, it can be easily prepared with a simple device called a gallium generator. In addition, because ⁶⁸Ga has superior positron-emitting capability, more clear images can be obtained as compared to when other radioisotopes or F18-FDG are used.

When ^{99m}Tc or ¹¹¹In is used as the metallic radioisotope, it is advantageous in that half-life is longer. And, ¹²³I, ¹²⁴I and ¹³¹I are advantageous in that manufacturing cost is decreased. And, when two or more radioisotopes of ⁶⁸Ga, ^{99m}Tc, ¹¹¹In, ¹⁸F, ¹¹C, ¹²³I, ¹²⁴I and ¹³¹I are attached at the same time, the effects of the respective radioisotopes are exerted together. For example, when ⁶⁸Ga and ^{99m}Tc are attached at the same time, it is advantageous in that diagnosis accuracy can be increased, application is possible even to a subject with diseases such as diabetes, application is possible even to the brain and heart, and half-life is increased.

The bifunctional chelating agent may be one or more selected from a group consisting of [1,4,7-triazacyclononane-1,4,7-triacetic acid (NOTA)], [1,4,7,10-tetraazacyclododecane-1,4,7,10-tetraacetic acid (DOTA)], diethylenetriaminepentaacetic acid (DTPA), hydrazinonicotinic acid (HYNIC), N₂S₂ and N₃S. Most specifically, it may be NOTA, although not being limited thereto. In particular, when NOTA is used as the bifunctional chelating agent, it is advantageous in that it is easily labeled with ⁶⁸Ga. Specifically, HYNIC, N₂S₂ or N₃S may be used when ^{99m}Tc is used as the label, and DOTA may be used when ¹¹¹In is used as the label.

The diagnosis method disclosed herein includes any diagnosis method known in the related art.

### Mode for Invention

Hereinafter, the present disclosure is described in detail through specific examples, so that those of ordinary skill in the art to which the present disclosure belongs can easily carry out the present disclosure. However, the present disclosure can be embodied in various different forms, without being limited to the examples.

### Examples

### Example 1: Preparation of NOTA-MSA for Ga-68 (⁶⁸Ga) labeling

### <Step 1: Preparation of phenyl mannose-bound human serum albumin>

After dissolving 20 mg of human serum albumin in 5 mL of a 0.1 M carbonate buffer (pH 9.5) and adding 5.5 mg of α-L-mannopyranosylphenyl isothiocyanate, reaction was conducted by stirring well at room temperature for 20 hours. Then, the reaction solution was stored at -70 ºC.

### <Step 2: Preparation of benzyl NOTA- and phenyl mannose-bound human serum albumin>

After adding 10 mg of p-SCN-Bz-NOTA to 1 mL of the mannosylated human serum albumin (13.6 mg/mL) prepared in the step 1, reaction was conducted at room temperature for 1 hour. After the reaction, benzyl NOTA- and phenyl mannose-bound human serum albumin was separated and purified using a Sephadex G-25 column.

### Example 2: Preparation of kit for imaging mannose receptor

After adding 1 mL of the benzyl NOTA- and phenyl mannose-bound human serum albumin (13.6 mg/mL) to 0.3 mL of a sodium acetate buffer (0.5 M, pH 5.5), and transferring to each vial an amount corresponding to 1 mg of protein, the mixture was freeze-dried and stored at -70 ºC.

### Example 3: Preparation of ⁶⁸Ga-labeled compound using kit for imaging mannose receptor

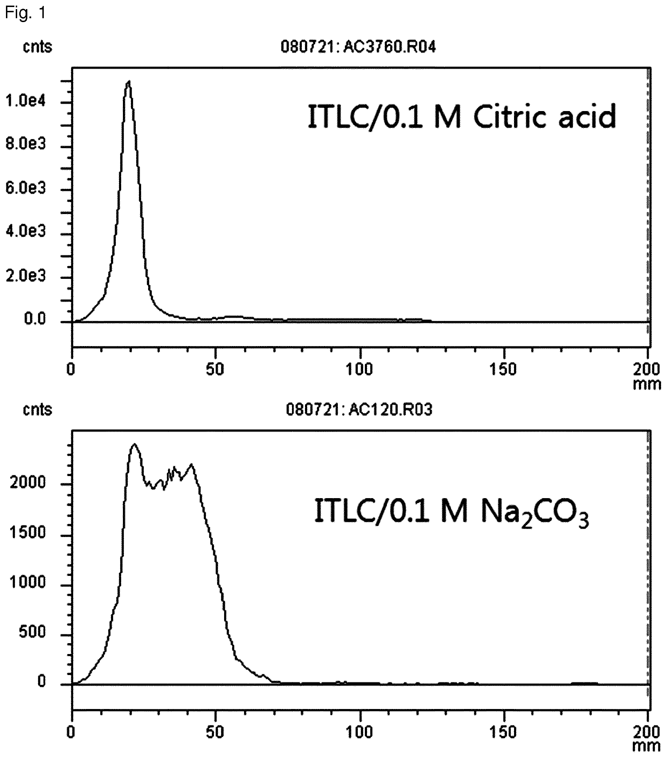

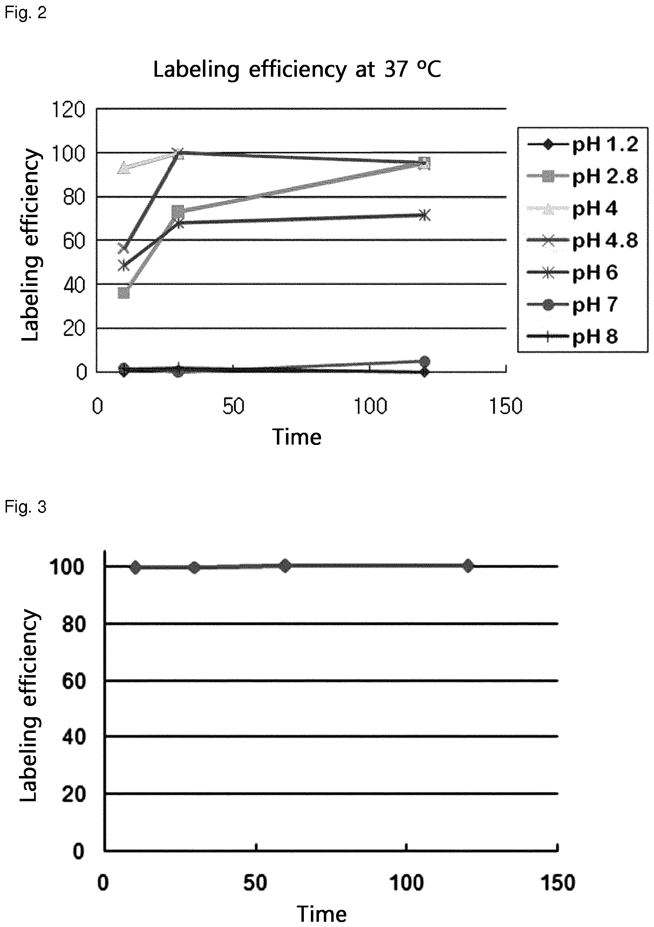

While conducting reaction at 37 ºC after adding 1 mL of a 0.1 M hydrochloric solution of ⁶⁸GaCl prepared using a ⁶⁸Ge/⁶⁸Ga generator (Cyclotron Co., Russia) to the kit of Example 2, labeling efficiency was measured by TLC 10 minutes, 30 minutes, 1 hour and 2 hours later. ITLC-SG (Gelman Co., USA) was used as a stationary phase and a 0.1 M citric acid solution was used as a mobile phase. The distribution of radioactivity on an ITLC plate was measured using a TLC scanner (Bioscan Co.). The labeled ⁶⁸Ga remained at the origin and unlabeled ⁶⁸Ga moved to the solvent front (Fig. 1). The labeling was almost completed after reaction at 37 ºC at pH 4-5 for 30 minutes (Fig. 2). The stability of the labeled ⁶⁸Ga-NOTA-MSA was investigated by measuring radiochemical purity after mixing with human serum and incubation at 37 ºC. The result is shown in Fig. 3. As can be seen from Fig. 3, when the compound was incubated in the serum at 37 ºC, the purity was maintained at 99% or higher for 2 hours. Considering that injection is made mostly within 1 hour after labeling in nuclear medical imaging, it can be seen that the compound is stable enough for practical purposes.

### Example 4: Preparation of RITC-MSA compound for fluorescence imaging of mannose receptor

First, MSA was prepared in the same manner as in the step 1 of Example 1. 100 mg of the MSA was reacted with 16 mg (0.03 mmol) of rhodamine B isothiocyanate (RITC) dissolved in 13 mL of a 0.1 M sodium carbonate buffer (pH 9.5) at room temperature for 20 hours in the dark. The produced RITC-MSA was separated and purified using a PD-10 column and physiological saline and then freeze-dried. The amount of RITC bound per MSA was calculated by measuring molecular weight using a MALDI-TOF mass spectrometer equipped with a nitrogen laser (337 nm). For this, the measurement was made by irradiating laser 500 times in a linear mode. All samples were analyzed 4 times and the molecular weight of MSA and RITC-MSA was determined by averaging the result.

A composition for imaging atherosclerosis was prepared through tis procedure.

PET/CT images were obtained for a patient with atherosclerotic symptoms using the prepared composition for imaging atherosclerosis. The PET/CT images were obtained using Philips' Extended Brilliance Workspace V3.5. Specifically, the imaging was conducted at Korea University Guro Hospital. The region of interest (ROI) of the aorta was selected such that the site of maximum radioactivity uptake was located at the center. For the adjacent slices in the axis direction of the ascending aorta and the descending aorta, the maximum standardized uptake value (SUV) and the mean standardized uptake value of the regions of interest were determined. The standardized uptake value was calculated by dividing the radioactivity concentration of the corresponding tissue by the whole body concentration of the injected radioactivity. The correlation coefficient of mean standardized uptake value between an inside observer and an outside observer was greater than 0.9.

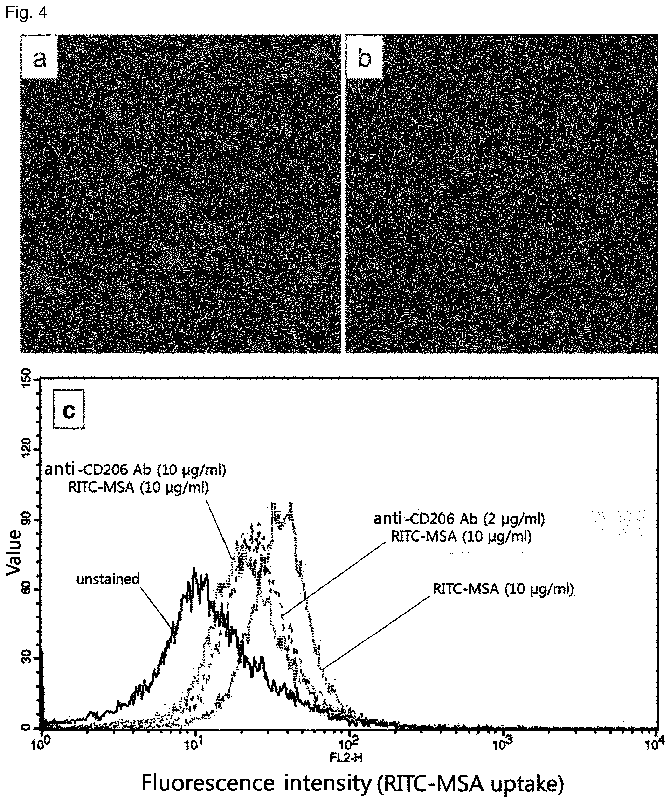

From Fig. 4, it can be seen that MSA-RITC is specifically bound to the mannose receptor in rat macrophages (Fig. 4a) and that the binding is inhibited upon pre-incubation with an anti-mannose receptor antibody (Fig. 4b). In addition, the flow cytometry analysis shows that the binding of MSA to the mannose receptor decreases in a content-dependent manner upon incubation with the anti-CD 206 anti-mannose receptor antibody (Fig. 4c).

### Additional Preparation Example: Preparation of Tc-99m-MSA (^{99m}Tc-MSA) using rMSA kit

After adding 2 mL or 5 mL of a physiological saline solution of pertechnetate (^{99m}TcO⁴⁻) prepared from a Mo-99/Tc-99m generator (Samyoung Unitech) to the kit of Example, reaction was conducted at room temperature for 1-30 minutes. The radioisotope labeling efficiency of technetium was determined by spotting a small amount of the reactant on an ITLC (instant thin layer chromatography) plate and then measuring the distribution of radioactivity using a TLC scanner after developing with physiological saline. The labeled technetium remained at the origin and all other unlabeled technetium moved to the solvent front. The labeling efficiency was 99% or higher. The stability of the labeled ^{99m}Tc-MSA was investigated by measuring radiochemical purity (%) with time when it was kept at room temperature and when it was mixed with human serum and then incubated at 37 ºC. The result is shown in Table 1.

**[Table 1]**

| Time (hr) | Serum | Room temperature |
|---|---|---|
| 0.5 | 96.0 | 94.5 |
| 1.0 | 92.2 | 96.4 |
| 2.0 | 96.0 | 91.1 |
| 3.0 | 94.4 | 94.0 |
| 6.0 | 97.4 | N.D. |
| 20.0 | 90.5 | 91.0 |
| 24.0 | 88.7 | 95.3 |

As seen from Table 1, the purity was maintained at 90% or higher for 20 hours both when the ^{99m}Tc-MSA was kept at room temperature and when it was incubated in serum at 37 ºC. Although the purity was decreased to 88.7% at 24 hours when it was incubated in serum at 37 ºC, it is stable enough for practical purposes because injection is made mostly within 1 hour after labeling in nuclear medical imaging.

### Comparative Example

In Comparative Example, F18-FDG, which has been used in a composition for imaging atherosclerosis, was used unlike Example.

### Test Examples

### Test Example 1: Molecular imaging of atherosclerosis in rabbit for Example

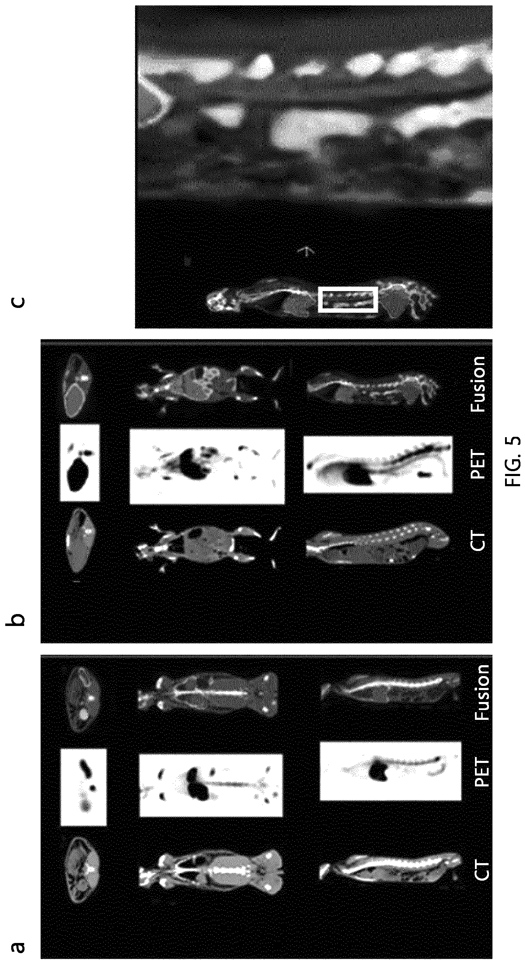

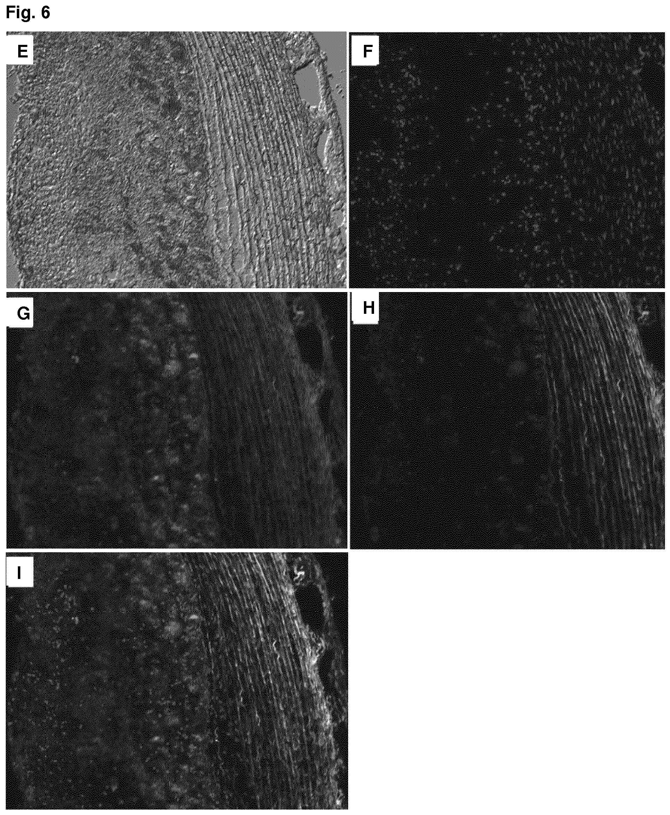

Ten 12-week-old normal rabbits (New Zealand White rabbits) were used for experiment. They were randomly divided into two groups of 5 rabbits. One group was given a normal diet and the other group was given a diet containing 1% cholesterol. The animals were kept under a standardized condition (21 ºC, 41-62% humidity) with regular light/dark (10/14 hr) cycles and were given free access to water and feed. 3 months later, after intravenously injecting 1 mCi of ⁶⁸Ga-MSA under anesthesia, positron emission tomography was performed on the whole body for 10 minutes from 10 minutes after the injection (Figs. 5 and 6). On the next day, the rabbits were anesthetized and RITC-MSA (42 µg/0.1 mL) or RITC mixed in 0.9% physiological saline was injected into the ear vein of the rabbits. 10 minutes later, the rabbits were euthanized and the aorta was cut and kept at -20 ºC. The aorta sections were fixed at room temperature for 30 minutes in a 4% (v/v) buffered formalin solution, washed with cold PBS (pH 7.4), embedded in optimum cutting temperature compound (OCT, Sakura, Tokyo) and kept at -80 ºC after a day for tissue penetration. Tissue slices cut to a thickness of 10 mm were mounted on slides together with poly-D-lysine and then kept at room temperature until use after drying at 45 ºC in the shade. The tissue slices were mounted using Fluoromount-G™ (SouthernBiotech, Birmingham, AL) after washing twice with PBS (pH 7.4). Fluorescence images were observed with an IX81-ZDC focus drift compensating microscope (Olympus, Tokyo, Japan) with excitation and emission wavelengths of 547 nm and 572 nm, respectively (Fig. 6).

Fig. 5a shows the PET/CT image of ⁶⁸Ga-MSA in the normal diet rabbit. It can be seen that atherosclerotic lesion is not observed and ⁶⁸Ga-MSA uptake has increased in liver and bone marrow tissues. Fig. 5b is the PET/CT image of ⁶⁸Ga-MSA in the cholesterol diet rabbit. Atherosclerotic lesion is observed in green color at the abdomen in front of the spine. Fig. 5c magnifies the atherosclerotic site in Fig. 5b. The green atherosclerotic lesion is observed in front of the yellow spine.

Fig. 6e shows the atherosclerotic tissue from the atherosclerotic site of the aorta of the cholesterol diet rabbit, observed by DIC (differential interference contrast) microscopy. Fig. 6f shows a result of observing the cell nucleus of the same tissue from the atherosclerotic lesion by fluorescence confocal microscopy after DAPI staining. Fig. 6g shows that the fluorescence of the RITC labeled at the MSA is observed at the atherosclerotic site. Fig. 6h shows that the fluorescence of RITC cannot be observed by fluorescence microscopy for the same atherosclerotic tissue when RITC not labeled at MSA was injected. Fig. 6i is an image obtained by superimposing the above images and shows the specific binding of RITC-MSA to the mannose receptor. It can be seen that the RITC-labeled MSA is bound well to the atherosclerotic site.

### Test Example 2: Comparison of atherosclerosis imaging for Example and Comparative Example

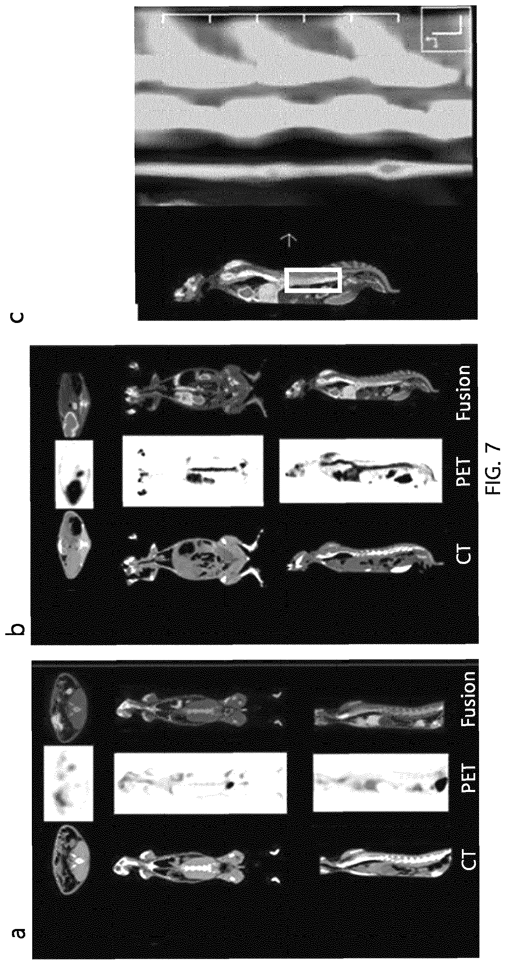

The diagnostic images of atherosclerosis was compared for Example and Comparative Example. Experiment was conducted by injecting ⁶⁸Ga-MSA and ¹⁸F-FDG to the same rabbits with 2-day intervals. The result is shown in Fig. 7, Fig. 8 and Fig. 9.

Fig. 7a shows the PET/CT image of ¹⁸F-FDG in the normal diet rabbit. It can be seen that atherosclerotic lesion is not observed and ¹⁸F-FDG uptake has increased in liver and bone marrow tissues. Fig. 7b is the PET/CT image of ⁶⁸Ga-MSA in the cholesterol diet rabbit. Atherosclerotic lesion is observed in green color at the abdomen in front of the spine. Fig. 7c magnifies the atherosclerotic site in Fig. 7b. The green atherosclerotic lesion is observed in front of the gray spine.

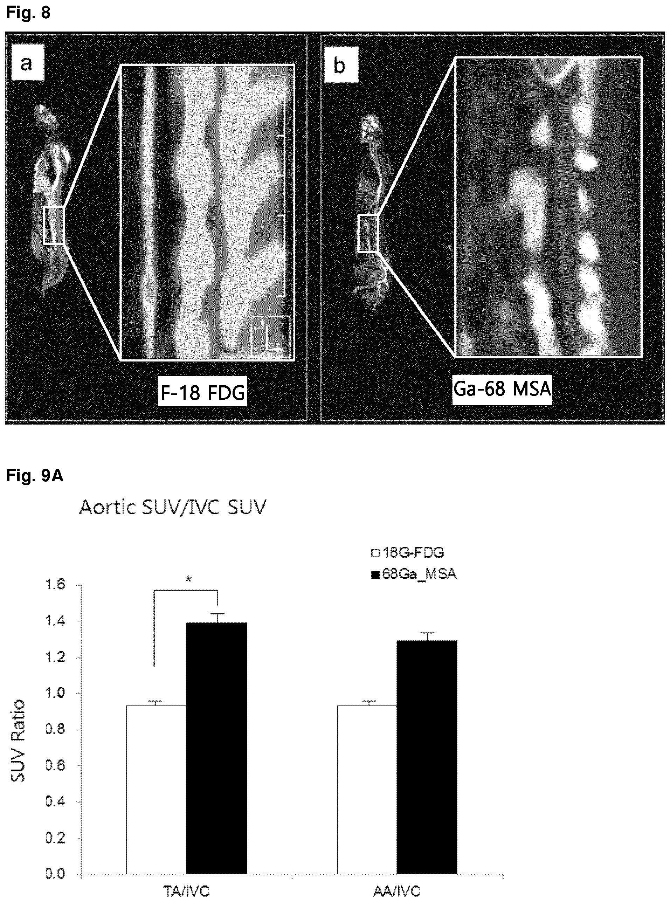

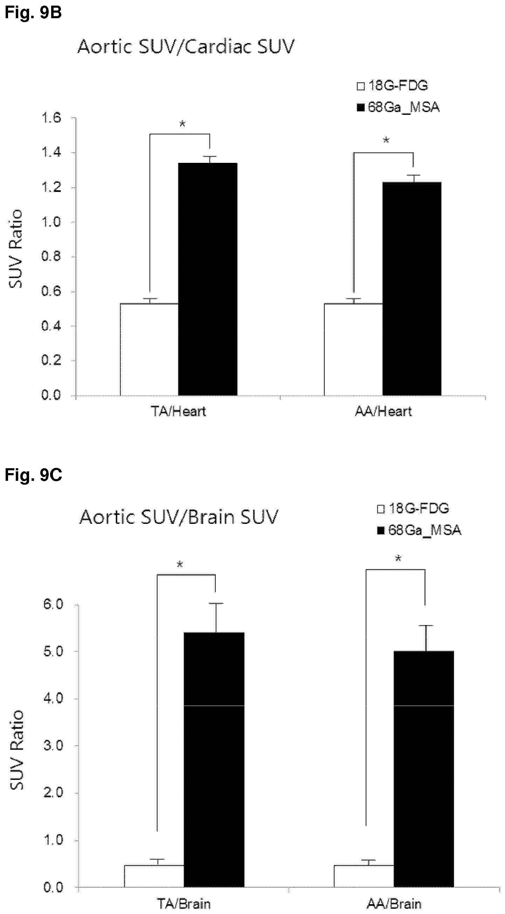

From Fig. 8, it can be seen that the atherosclerotic lesion can be observed with both ¹⁸F-FDG and ⁶⁸Ga-MSA for Comparative Example. Meanwhile, in the increase in uptake, a better result was obtained for Example wherein ⁶⁸Ga-MSA was used than for Comparative Example wherein ¹⁸F-FDG was used, as seen from Fig. 9. More specifically, Fig. 9a shows the SUV of the inferior vena cava relative to the SUV of the aortic atherosclerotic lesion (TBR: target-to-background ratio), Fig. 9b shows the TBR of the cardiac SUV relative to the SUV of the aortic atherosclerotic lesion, and Fig. 9c shows the TBR of the brain parenchymal SUV relative to the SUV of the aortic atherosclerotic lesion. From Fig. 9, it can be seen that a better result was achieved for Example than for Comparative Example. Accordingly, it was confirmed that clearer atherosclerosis imaging can be achieved for Example than for Comparative Example.

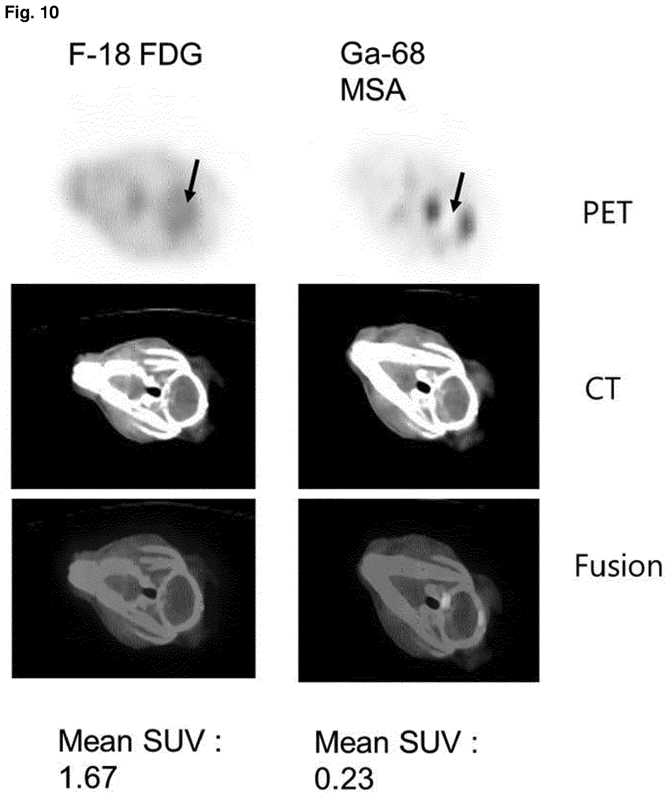

Table 2 shows the TBR of the SUV of the aortic atherosclerotic site relative to the brain SUV. It can be seen that the TBR is higher for Example than for Comparative Example. Fig. 10 shows that the effect of the brain SUV in PET is very low for Example as compared to Comparative Example. In contrast, for Comparative Example, because ¹⁸F-FDG enters the brain parenchyma through the blood-brain barrier, it is not certain whether the signal originates from the atherosclerotic lesion or from the increase in ¹⁸F-FDG. Accordingly, it can be seen that ¹⁸F-FDG is not useful in detecting atherosclerotic lesions in the brain tissue.

**[Table 2]**

| | Organ | ¹⁸F-FDG | ⁶⁸Ga-MSA | p value |
|---|---|---|---|---|
| SUV | Brain | 1.92 ± 0.55 | 0.40 ± 0.17 | 0.027 |
| | Heart | 1.58 ± 0.33 | 1.52 ± 0.40 | 0.674 |
| | Liver | 1.80 ± 0.50 | 10.21 ± 2.36 | 0.028 |
| | Spleen | 0.97 ± 0.41 | 4.07 ± 1.59 | 0.028 |
| | Bone marrow | 0.78 ± 0.24 | 1.97 ± 0.78 | 0.028 |
| | Thoracic aorta | 0.96 ± 0.94 | 2.03 ± 0.67 | 0.028 |
| | Abdominal aorta | 0.88 ± 0.88 | 1.73 ± 0.55 | 0.028 |
| | IVC | 0.96 ± 0.37 | 1.53 ± 0.63 | 0.027 |
| TBR of SUV | Thoracic aorta/IVC | 0.93 ± 0.16 | 1.39 ± 0.34 | 0.046 |
| | Abdominal aorta/IVC | 0.93 ± 0.27 | 1.29 ± 0.61 | 0.249 |
| | Thoracic aorta/heart | 0.53 ± 0.10 | 1.34 ± 0.19 | 0.028 |
| | Abdominal aorta/heart | 0.53 ± 0.10 | 1.23 ± 0.53 | 0.046 |
| | Thoracic aorta/brain | 0.46 ± 0.09 | 5.41 ± 1.66 | 0.028 |
| | Abdominal aorta/brain | 0.46 ± 0.13 | 5.00 ± 2.85 | 0.028 |

### Test Example 3: Evaluation of clinical applicable for Example

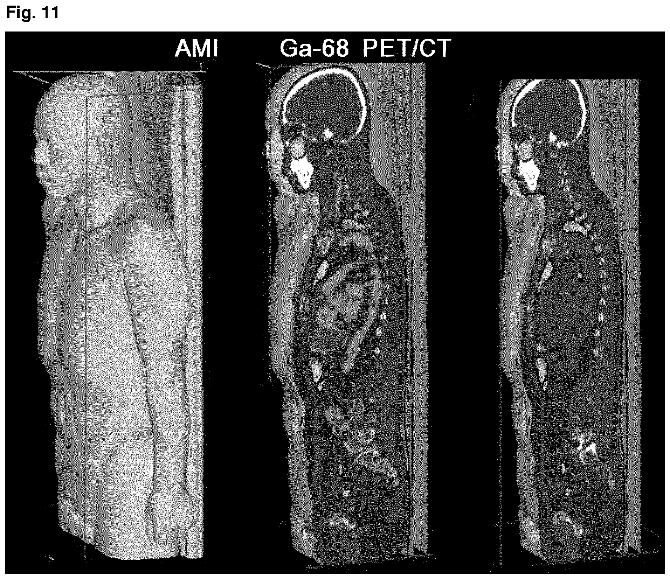

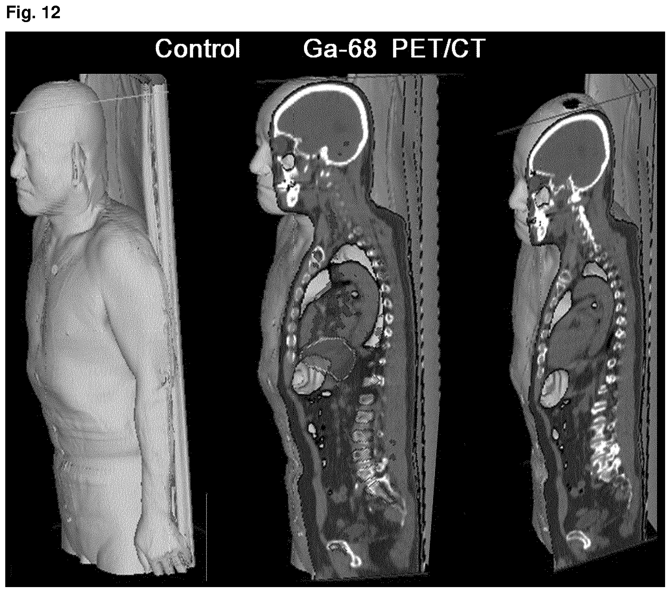

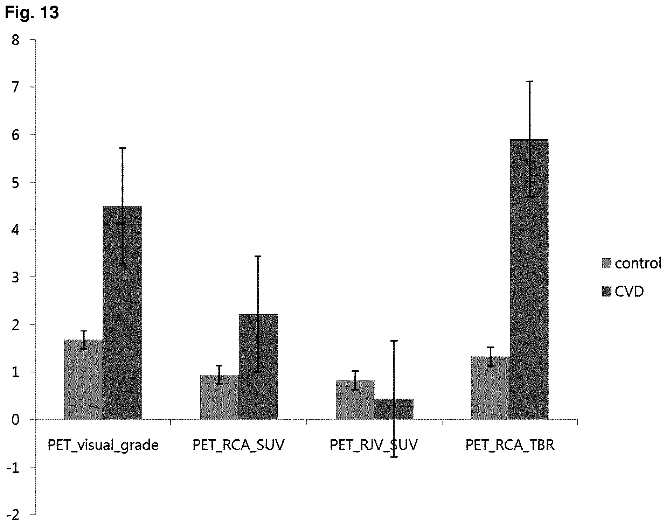

Experiment was conducted to compare clinical applicability of the compound of Example as compared to Comparative Example. A phase I clinical study of comparing ⁶⁸Ga-MSA images of carotid atherosclerotic sites for an acute myocardial infarction patient group and a normal control group was conducted. The result is shown in Fig. 11, Fig. 12 and Fig. 13. Fig. 11 shows the ⁶⁸Ga-MSA image of an acute myocardial infarction patient at the carotid atherosclerotic lesion site. The green atherosclerotic site is observed well in the neck area. Fig. 12 shows the ⁶⁸Ga-MSA image of the control group with no atherosclerotic lesion at the carotid. It can be seen that no abnormal site is observed. From Fig. 13, it can be seen that the presence or absence of atherosclerotic lesions in cardiovascular disease patients having atherosclerosis at the carotid can be easily detected from visual inspection (PET_visual_grade), SVU (PET_RCA_grade) and target SUV/background SUV ratio (PET_RCA_TBR) of ⁶⁸Ga-MSA PET/CT images as compared to the normal control group. Accordingly, it was confirmed that the composition for imaging atherosclerosis of Example is clinically applicable.

## Claims

1. A composition for use in a method of in vivo diagnosis of atherosclerosis, which composition comprises a radioisotope-labeled compound which is a bifunctional chelating agent-mannosylated human serum albumin, labeled with a radioisotope.

2. The composition for use in a method of in vivo diagnosis of atherosclerosis according to claim 1, wherein the radioisotope is one or more selected from a group consisting of ⁶⁸Ga, ^{99m}Tc, ¹¹¹In, ¹⁸F, ¹¹C, ¹²³I, ¹²⁴I and ¹³¹I.

3. The composition for use in a method of in vivo diagnosis of atherosclerosis according to claim 1, wherein the radioisotope is ⁶⁸Ga.

4. The composition for use in a method of in vivo diagnosis of atherosclerosis according to claim 1, wherein the bifunctional chelating agent is one or more selected from a group consisting of [1,4,7-triazacyclononane-1,4,7-triacetic acid (NOTA)], [1,4,7,10-tetraazacyclododecane-1,4,7,10-tetraacetic acid (DOTA)], diethylenetriaminepentaacetic acid (DTPA), hydrazinonicotinic acid (HYNIC), N₂S₂ and N₃S.

5. The composition for use in a method of in vivo diagnosis of atherosclerosis according to claim 1, wherein the bifunctional chelating agent is 1,4,7-triazacyclononane-1,4,7-triacetic acid (NOTA).

6. A method for imaging atherosclerosis using the composition as defined in any of claims 1 to 5.

## Patentansprüche

1. Zusammensetzung zur Verwendung in einem Verfahren von In-vivo-Diagnose von Atherosklerose, wobei die Zusammensetzung eine Radioisotop-markierte Verbindung umfasst, die ein bifunktionelles chelatbildendes, Wirkstoffmannosyliertes humanes Serumalbumin ist, markiert mit einem Radioisotop.

2. Zusammensetzung zur Verwendung in einem Verfahren von In-vivo-Diagnose von Atherosklerose nach Anspruch 1, wobei das Radioisotop eines oder mehrere, ausgewählt aus einer Gruppe, bestehend aus ⁶⁸Ga, ^{99m}Tc, ¹¹¹In, ¹⁸F, ¹¹C, ¹²³I, ¹²⁴I und ¹³¹I, ist.

3. Zusammensetzung zur Verwendung in einem Verfahren von In-vivo-Diagnose von Atherosklerose nach Anspruch 1, wobei das Radioisotop ⁶⁸Ga ist.

4. Zusammensetzung zur Verwendung in einem Verfahren von In-vivo-Diagnose von Atherosklerose nach Anspruch 1, wobei der bifunktionelle chelatbildende Wirkstoff eines oder mehrere, ausgewählt aus einer Gruppe bestehend aus [1,4,7-Triazacyclononan-1,4,7-Triessigsäure (NOTA)], [1,4,7,10-Tetraazacyclododecan-1,4,7,10-Tetraessigsäure (DOTA)], Diethylentriaminpentaessigsäure (DTPA), Hydrazin-Nikotinsäure (HYNIC), N₂S₂ und N₃S, ist.

5. Zusammensetzung zur Verwendung in einem Verfahren von In-vivo-Diagnose von Atherosklerose nach Anspruch 1, wobei der bifunktionelle chelatbildende Wirkstoff 1,4,7-Triazacyclononan-1,4,7-Triessigsäure (NOTA) ist.

6. Verfahren zum Abbilden von Atherosklerose unter Verwendung der Zusammensetzung wie in einem der Ansprüche 1 bis 5 definiert.

## Revendications

1. Composition pour une utilisation dans une méthode de diagnostic in vivo de l'athérosclérose, laquelle composition comprend un composé marqué par un isotope radioactif qui est un composé d'agent chélatant bifonctionnel et de sérumalbumine humaine mannosylée, marqué par un isotope radioactif.

2. Composition pour une utilisation dans une méthode de diagnostic in vivo de l'athérosclérose selon la revendication 1, dans laquelle l'isotope radioactif est un ou plusieurs choisis dans le groupe constitué par ⁶⁸Ga, ^{99m}Tc, ¹¹¹In, ¹⁸F, ¹¹C, ¹²³I, ¹²⁴I et ¹³¹I.

3. Composition pour une utilisation dans une méthode de diagnostic in vivo de l'athérosclérose selon la revendication 1, dans laquelle l'isotope radioactif est ⁶⁸Ga.

4. Composition pour une utilisation dans une méthode de diagnostic in vivo de l'athérosclérose selon la revendication 1, dans laquelle l'agent chélatant bifonctionnel est un ou plusieurs choisis dans le groupe constitué par l'acide 1,4,7-triazacyclononane-1,4,7-triacétique (NOTA), l'acide 1,4,7,10-tétraazacyclododécane-1,4,7,10-tétraacétique (DOTA), l'acide diéthylènetriaminepentaacétique (DTPA), l'acide hydrazinonicotinique (HYNIC), N₂S₂ et N₃S.

5. Composition pour une utilisation dans une méthode de diagnostic in vivo de l'athérosclérose selon la revendication 1, dans laquelle l'agent chélatant bifonctionnel est l'acide 1,4,7-triazacyclononane-1,4,7-triacétique (NOTA).

6. Méthode d'imagerie de l'athérosclérose utilisant la composition telle que définie selon l'une quelconque des revendications 1 à 5.
